# EUROPEAN PATENT APPLICATION

(11) **EP 1 277 465 A1**
(43) Date of publication of application: **22.01.2003**
(21) Application number: 01921993.0
(22) Date of filing: 23.04.2001
(51) Int. Cl.: A61K 9/50, A61K 47/34, B01J 13/12, C08J 3/16

(54) **PROCESS FOR PRODUCING MICROSPHERE**

(30) Priority: 24.04.2000 JP 2000122469
(71) Applicant: TANABE SEIYAKU CO., LTD., Chuo-ku, Osaka-shi, Osaka 541-8505 (JP)
(72) Inventor: SUZUKI, Takehiko, Toyono-gun, Osaka 563-0104 (JP); KITAZAWA, Takeo, Ichikawa-shi, Chiba 272-0034 (JP); MATSUMOTO, Akihiro, Hirakata-shi, Osaka 573-0064 (JP); SUZUKI, Akira, Itami-shi, Hyogo 664-0017 (JP)
(74) Representative: HOFFMANN EITLE
(86) International application number: JP0103446
(87) International publication number: WO01080835

(57) **Abstract**

A process for producing microspheres, which comprises adding a polymer solution containing a medicament, a biodegradable polymer and a good solvent for said polymer which solvent is miscible with water (Solvent A) to a homogeneous mixture of a poor solvent for said polymer which solvent is miscible with said Solvent A (Solvent B) and a poor solvent for said polymer which solvent is immiscible with said Solvent A (Solvent C), emulsifying the mixture to prepare an emulsion wherein the polymer solution forms a dispersed phase and the homogeneous mixture forms a continuous phase, and then removing Solvent A from the dispersed phase.

## Description

### TECHNICAL FIELD

This invention relates to a process for producing microspheres using a water-miscible organic solvent

### BACKGROUND ART

Medicinal treatments for a longer period is possible by only one administration through a subcutaneous route, intramuscular route, etc. of a microsphere preparation containing a medicament in a biodegradable polymer which is hardly soluble in water, e.g., polylactic acid, poly(lactic-co-glycolic acid).

Such microsphere preparations are prepared, for example, by dissolving or dispersing a medicament in a solution of a polymer in an organic solvent, emulsifying it in an aqueous phase, removing the organic solvent in the aqueous phase, and solidifying the polymer (JP-A-61-63613, JP-A-63-122620, JP-A-04-46116, etc.).

According to this method, it is said that it is important to use an organic solvent capable of dissolving said polymer but which solvent being immiscible with water, and hence, halogenated aliphatic hydrocarbon solvents (e.g., dichloromethane, chloroform, etc.) are generally used as the organic solvent.

These solvents are however toxic to human body and residual amount in microspheres are going to be regulated (agreed in the Japan-US-EU International Conference on Harmonization of Technical Requirements for Registration of Pharmaceuticals for Human Use (ICH); "A Guideline for Residual Solvents in Medicaments" published on March 30, 1998). Since halogenated aliphatic solvents have the risk of depletion of ozone layer, or the risk of acting as an environment hormone, the leaking of these solvents from production line to environment will also more strictly be regulated (e.g., the Act for Promotion of Improvement in Out-put to Environment and Management of Specific Chemicals issued on July 13, 1999; as well as its enforcement ordinance issued on March 29, 2000).

On the other hand, Japanese Patent No. 2,564,386, and Drug Development and Industrial Pharmacy, 24(12): 1113-1128 (1998) disclose a method for producing nanospheres by dissolving a polymer in acetone which is miscible with water and is less harmful to human body and environment; adding to the polymer solution an aqueous solution of a solute (e.g., an inorganic electrolyte) in a high concentration for subjecting the mixture to phase inversion to give an O/W emulsion; and adding water to this emulsion for extracting acetone.

This method however uses only water as a solvent for emulsifying the polymer solution, and the solute to be dissolved in water is merely an inorganic base for salting out. Furthermore, it is disclosed that this method can be applicable only to a medicament being fat-soluble.

Besides, Japanese Patent No. 2,608,242 discloses a method for preparing microspheres by dispersing an O/W emulsion in an aqueous solution of saccharides, etc. to prepare a W/O/W emulsion, and subjecting it to an in-water drying. The organic solvent for this O/W emulsion is a water-immiscible solvent such as methylene chloride, etc. The saccharides etc. added to water is for controlling osmotic pressure to prevent leaking of a water-soluble medicament from the inner aqueous phase.

### DISCLOSURE OF INVENTION

The present invention provides a process for producing microspheres using a water-miscible organic solvent, which is applicable to either water-soluble or fat-soluble medicaments. More specifically, the present invention relates to a process for producing microspheres using a water-miscible organic solvent, e.g., acetone, tetrahydrofuran, etc., being less harmful to human body and environment.

After repeated investigations, the present inventors have found that microspheres having superior characteristics can be prepared efficiently by using a combination of the following (1) and (2) solvents. Further, they have found that this process can be applicable to both of water-soluble and fat-soluble medicaments, and then the present invention has been completed.
(1) A water-miscible organic solvent which dissolves a biodegradable polymer (i.e., a good solvent for said polymer which solvent being miscible with water: which is referred to as "Solvent A"):
(2) A homogeneous mixture of a solvent which hardly dissolves the above polymer but is miscible with Solvent A (i.e., a poor solvent for said polymer which solvent is miscible with Solvent A: which is referred to as "Solvent B") and a solvent which hardly dissolves the above polymer but is immiscible with Solvent A (i.e., a poor solvent for said polymer which solvent is immiscible with Solvent A: which is referred to as "Solvent C"):

Thus, the present invention relates to a process for producing microspheres, which is characterized by adding a polymer solution containing a medicament, a biodegradable polymer and Solvent A into a homogeneous mixture composed of Solvent B and Solvent C to afford an emulsion in which the polymer solution forms a dispersed phase and the homogeneous mixture forms a continuous phase; and then removing Solvent A from the dispersed phase.

### BRIEF DESCRIPTION OF DRAWINGS

Figure 1 is a graph showing a comparison of dissolution patterns of vitamin B₁₂ from microspheres prepared by adding a polymer solution into a homogeneous solution (Example 1) and those from microspheres prepared by adding a homogeneous solution into a polymer solution (Comparative Example 1).
Figure 2 is a graph showing the results of the dissolution test *in vitro* of microspheres containing vitamin B₁₂ (Example 2).
Figure 3 is a graph showing the results of the dissolution test *in vitro* of microspheres containing bovine serum albumin (Example 7).
Figure 4 is a graph showing the results of the dissolution test *in vitro* of microspheres containing taltirelin (Example 8).
Figure 5 is a graph showing the results of the dissolution test *in vitro* of microspheres containing vitamin B₁₂ (Example 9).

### BEST MODE FOR CARRYING OUT THE INVENTION

In carrying out the present invention, a polymer solution is at first prepared to contain a medicament, a biodegradable polymer, and a good solvent for said polymer, which solvent is water-miscible (Solvent A).

There is no specific limitation for the medicaments, and either water-soluble or fat-soluble one may preferably be used.

Specific examples of the medicaments include, but not limited thereto, anti-tumor agents, physiologically active peptides, antibiotics, anti-pyretics, analgesics, antiinflammatories, antitussives, expectorants, sedatives, muscle relaxants, antiepileptics, antiulcers, antidepressants, antiallergic agents, cardiotonics, antiarrythmic agents, vasodilators, antihypertensive diuretics, antidiabetics, antilipemic agents, anticoagulants, hemostatics, antitubercular agents, hormones, antinarcotic agents, bone resorption inhibitors, promoters of osteogenesis, antiangiogenetics, antiemetics, vitamins, etc.

Antitumor agents include, for example, paclitaxel, bleomycin, methotrexate, actinomycin D, mitomycin C, vinblastine sulfate, vincristine sulfate, daunorubicin, doxorubicin, neocarcinostatin, cytosine arabinoside, fluorouracil, tetrahydrofuryl-5-fluorouracil, krestin, picibanil, lentinan, tamoxifen, levamisole, bestatin, azimexon, glycyrrhizin, cisplatin, carboplatin, irinotecan hydrochloride, etc

Physiologically active peptides include, for example, insulin, somatostatin, sandostatin, growth hormone, prolactin, adrenocortotropic hormone (ACTH), ACTH derivatives, melanocyte stimulating hormone (MSH), thyrotrophin releasing hormone (TRH) and its derivatives (e.g., taltirelin, etc.), thyroid stimulating hormone (TSH), luteinizing hormone (LH), luteinizing hormone releasing hormone (LHRH) and its derivatives (e.g., leuprorelin acetate, etc.), follicle stimulating hormone (FSH), vasopressin, desmopressin, oxytocin, calcitonin, elcatonin, parathyroid hormone (PTH), glucagons, gastrin, secretin, pancreozymin, cholecystokinin, angiotensin, human placental lactogen, human chorionic gonadotropin (HCG), enkephalin, enkephalin derivatives, endorphin, kyotorphin, interferons (e.g., α-, β-, γ-, etc.), interleukins (e.g., 1, 2, 3, 4, 5, 6, 7, 8, 9, 10, 11, 12, etc.), taftsin, thymopoietin, thymosin, thymostimulin, thymic humoral factor (THF), serum thymic factor (FTS) and its derivatives, and other thymic factors, tumor necrosis factor (TNF), chemokines and its derivatives, minicytokines and its derivatives, colony stimulating factors (e.g., CSF, GCSF, GMCSF, MCSF), motilin, dinorphin, bombesin, neurotensin, cerulein, bradykinin, urokinase, asparaginase, kallikrein, substance P, insulin-like growth factor (IGF-I, IGF-II), nerve growth factor (NGF), cell growth factors (e.g., EGF, TGF-α, TGF-β, PDGF, FGF hydrochloride, basic FGF, etc.), bone morphogenetic protein (BMP), neurotrophic factors (e.g., NT-3, NT-4, CNTF, GDNF, BDNF, etc.), blood coagulation factors VIII and IX, lysozyme chloride, polymixin B, colistin, gramicidin, bacitracin, erythropoietin (EPO), thrombopoietin (TPO), etc.

The antibiotics include, for example, gentamycin, dibekacin, kanendomycin, lividomycin, tobramycin, amikacin, fradiomycin, sisomicin, tetracycline hydrochloride, oxytetracycline hydrochloride, rolitetracycline, doxycycline hydrochloride, ampicillin, piperacillin, ticarcillin, aspoxycillin, cephalothin, cephaloridine, cefotiam, cefsulodin, cefmenoxime, cefmethazole, cefazolin, cefotaxime, cefoperazone, ceftizoxime, moxolactam, thienamycin, sulfazecin, azthreonam, etc.

The antipyretics, analgesics and anti-inflammatory agents include, for example, salicylic acid, sulpyrine, flufenamic acid, diclofenac, indomethacin, morphine, pethidine hydrochloride, levorphanol tartrate, oxymorphone, etc.

The antitussives and expectorants include, for example, ephedrine hydrochloride, methylephedrine hydrochloride, noscapine hydrochloride, codeine phosphate, dihydrocodeine phosphate, alloclamide hydrochloride, chlophedianol hydrochloride, picoperidamine hydrochloride, cloperastine, protokyrol hydrochloride, isoproterenol hydrochloride, salbutamol sulfate, terbutaline sulfate, etc.

The sedatives include, for example, chlorpromazine, prochlorperazine, trifluoperazine, atropine sulfate, methscopolamine bromide, etc.

The muscle relaxants include, for example, pridinol methanesulfonate, tubocurarine chloride, pancuronium bromide, etc.

The anti-epileptics include, for example, phenytoin, ethosuximide, sodium acetazolamide, chlordiazepoxide, etc.

The antiulcers include, for example, metoclopramide, histidine hydrochloride, etc.

The antidepressants include, for example, imipramine, clomipramine, noxiptiline, phenelzine sulfate, etc.

The antiallergic agents include, for example, diphenhydramine hydrochloride, chlorpheniramine maleate, tripelenamine hydrochloride, methdilazine hydrochloride, clemizol hydrochloride, diphenylpyraline hydrochloride, methoxyphenamine hydrochloride, etc.

The cardiotonics include, for example, trans-π-oxocamphor, theophylol, aminophylline, etilefrine hydrochloride, etc.

The anti-arrythmia agents include, for example, azimilide, propranolol, alprenolol, bufetorol, oxyprenolol, etc.

The vasodilators include, for example, oxyfedrine hydrochloride, diltiazem hydrochloride, tolazoline hydrochloride, hexobendine, bamethan sulfate, etc.

The antihypertensive diuretics include, for example, hexamethonium bromide, pentrilium, mecamylamine hydrochloride, ecarazine hydrochloride, clonidine, etc.

The anti-diabetics include, for example, glymidine sodium, glypizide, phenformin hydrochloride, buformin hydrochloride, metformin, etc.

The anti-hyperlipidemic agents include, for example, mevalotin, pravastatin sodium, simvastatiri, clinofibrate, clofibrate, simfibrate, bezafibrate, etc.

The anticoagulants include, for example, heparin sodium, etc.

The hemostatics include, for example, thromboplastin, thrombin, menadione sodium bisulfite, acetomenaphthone, ε-aminocaproic acid, tranexamic acid, carbazochrome sodium sulfonate, adrenochrome monoaminoguanidine methanesulfonate, etc.

The anti-tubercular agents include, for example, isoniazid, ethambutol, p-aminosalicylic acid, etc.

The hormones include, for example, prednisolone, prednisolon sodium phosphate, dexamethasone sodium hydrochloride, hexestrol phosphate, methimazole, estrone, etc.

The antinarcotic agents include, for example, levallorphan tartrate, nalorphine hydrochloride, naloxone hydrochloride, etc.

The bone resorption inhibitors include, for example, ipriflavone, etc.

The promoters of osteogenesis include, for example, polypeptides such as BMP, PTH, TGF-β, IGF-I, etc.

The antiangiogenetics include, for example, angiogenesis suppressing steroids, fumagillin, fumagillol derivatives, angiostatin, endostatin, etc.

The antiemetics include, for example, 5-hydroxytryptamine type 3 receptor antagonists such as ondansetron or tropisetron, neurokinin 1 receptor antagonists, etc.

Vitamins includes, for example, vitamin A, β-carotene, vitamin B₁, vitamin B₂, niacin, nicotinamide, pantothenic acid, calcium pantothenate, vitamin B₆, vitamin B₁₂, folic acid, inositol, para-amino-hippuric acid, biotin, vitamin C, vitamin D, vitamin E, vitamin K, etc.

The medicaments referred to the above may be in free form or be in a pharmaceutically acceptable salt form. For example, when the medicament possesses a basic group such as an amino group, etc., it may be used in the form of a salt with an inorganic acid (e.g., hydrochloric acid, sulfuric acid, nitric acid, etc.) or with an organic acid (e.g., carbonic acid, succinic acid, etc.). When the medicament possesses an acidic group such as a carboxyl group, it may be used in the form of a salt with an inorganic base (e.g., alkali metals such as sodium, potassium, etc.) or with an organic base (e.g., organic amines such as triethylamine, basic amino acids such as arginine, etc.).

When the encapsulation efficiency of a medicament into microspheres is low due to its salt formation, salts may be converted into the free form. Conversion into free form may be carried out for an acid-addition salt, by treating with a basic aqueous solution (e.g., an aqueous alkali metal hydrogen carbonate solution, an aqueous alkali metal carbonate solution, an alkali metal hydroxide, an alkali metal phosphate, an aqueous alkali metal hydrogen phosphate solution, a weakly basic buffer solution, etc.), followed by extraction with an organic solvent; or for a base-addition salt, by treating with a weakly acidic aqueous solution (e.g., an aqueous ammonium chloride solution, a weakly acidic buffer solution, etc.), followed by extraction with an organic solvent. The medicament in the free form may be recovered from the extract by removing the solvent by a usual method.

The concentration of medicament in polymer solution may be 0.001 - 90 (w/w) %, preferably 0.01 - 50 (w/w) %.

In the polymer solution, the medicament may be either in a dissolved or dispersed state. In a dispersed state, the medicament is preferably made in fine particles in advance. Fine particles may be obtained by using a conventional method, such as pulverization, crystallization, spray drying, etc.

For pulverization, the medicament may physically be pulverized by using a pulverizer, for example, jet-mill, hammer mill, rotary ball-mill, vibratory ball-mill, beads mill, shaker mill, rod mill, tube mill, etc.

For crystallization, the medicament may firstly be dissolved in an appropriate solvent; the resulting solution is subjected to adjusting pH, controlling temperature, altering of solvent composition, etc. to precipitate crystals; and then the precipitated crystals are recovered by a method such as filtration, centrifugation, etc.

For spray-drying, the medicament may be dissolved in a suitable solvent; the resulting solution is sprayed into a drying chamber of a spray dryer apparatus using a spray nozzle; and the solvent in spray droplets is evaporated in a very short time.

When the medicament is a polypeptide, it may also be made in fine particles by the following steps. A mixed aqueous solution of a polypeptide and a polyethylene glycol may be lyophilized, and the resulting cake is treated with a solvent in which the polypeptide is insoluble but polyethylene glycol is soluble (cf., JP-A-11-302156).

The biodegradable polymer may be any one conventionally used in pharmaceutical field, and especially preferable ones are polyesters of hydroxyfatty acids. The average molecular weight of said polyester of hydroxyfatty acid may preferably be about 2,000 to about 800,000, more preferably about 5,000 to about 200,000.

Among the above polyesters of hydroxyfatty acids, more preferable ones are polylactic acid, poly(lactic-co-glycolic acid), poly(2-hydroxybutyric-co-glycolic acid). The poly(lactic-co-glycolic acid) has preferably a molar ratio of lactic acid/glycolic acid in the range of 90/10 to 30/70, more preferably 80/20 to 40/60, and the poly(2-hydroxy-butyric-co-glycolic acid) has preferably a molar ratio of 2-hydroxy-butyric acid/glycolic acid in the range of 90/10 to 30/70, more preferably 80/20 to 40/60.

In the polymer solution, the concentration of a biodegradable polymer may vary depending upon the kinds and molecular weight of the polymer, but it is usually in the range of 1 to 80 % by weight, preferably 20 to 60 % by weight.

The good solvent for the biodegradable polymer which solvent is miscible with water (Solvent A) is not specifically limited as far as the amount of the solvent being necessary to dissolve 1 g of the biodegradable polymer is less than 25 g and it is completely miscible with water. Preferable examples are acetone, tetrahydrofuran, acetonitrile, dimethylformamide, dimethylsulfoxide, dioxane, diglyme, ethylene glycol dimethyl ether, etc. Among these, acetone and tetrahydrofuran are preferable from the viewpoint of less toxicity, and acetone is most preferable. These solvents may be used as a sole solvent or as a mixture of two or more thereof.

The polymer solution may be prepared by dissolving a biodegradable polymer in Solvent A, and dissolving or dispersing a medicament therein. There is no specific limitation in addition sequence of a medicament and a biodegradable polymer.

Further, the polymer solution may contain a small amount of a poor solvent for the biodegradable polymer which solvent is miscible with Solvent A (Solvent B).

The amount of Solvent B in the polymer solution is preferably in such a range so as not to precipitate the biodegradable polymer, for example, in the range of 0.001 to 50 % by weight, preferably 0.01 to 20 % by weight.

Then, the resulting polymer solution is added to a homogeneous mixture containing a poor solvent for said biodegradable polymer which solvent is miscible with Solvent A (Solvent B) and a poor solvent for said biodegradable polymer which solvent is immiscible with Solvent A (Solvent C) to afford an emulsion in which the polymer solution forms a dispersed phase and the homogeneous mixture forms a continuous phase.

Solvent B is not specifically limited as far as the amount of the solvent being necessary to dissolve 1 g of the biodegradable polymer is 25 g or more and it is completely miscible with Solvent A. Specific examples include water and a monovalent alcohol having 1 to 4 carbon atoms. Specific examples of the alcohol are methanol, ethanol, n-propanol, isopropanol, n-butanol, isobutanol, sec-butanol, tert-butanol, etc. Among these, preferable one are water and ethanol, and most preferable one is water.

Besides, Solvent C is no specifically limited as far as the amount of the solvent being necessary to dissolve 1 g of the biodegradable polymer is 25 g or more, and the amount of Solvent A being miscible with 100 parts by weight of Solvent C is not more than 25 parts by weight, and it forms a completely homogeneous mixture with Solvent B. Specific example of Solvent C is glycerin.

Some examples of preferable combination of Solvents A, B and C are, while not limited thereto, a combination of acetone as Solvent A, water as Solvent B and glycerin as Solvent C; a combination of acetone as Solvent A, ethanol as Solvent B and glycerin as Solvent C; a combination of tetrahydrofuran as Solvent A, water as Solvent B and glycerin as Solvent C; a combination of acetone as Solvent A, a mixture of water and ethanol as Solvent B and glycerin as Solvent C; a combination of acetone as Solvent A, n-propanol as Solvent B and glycerin as Solvent C; a combination of acetone as Solvent A, n-butanol as Solvent B and glycerin as Solvent C; and a combination of acetone as Solvent A, isopropanol as Solvent B and glycerin as Solvent C, etc. Among these combinations, the most preferable one is a combination of acetone as Solvent A, water as Solvent B and glycerin as Solvent C.

The ratio of Solvent B/Solvent C by weight in the homogeneous mixture varies depending upon the kinds of a biodegradable polymer, the selected combination of Solvents A, B and C, etc., but is preferably in the range of 5:95 to 75:25 by weight for giving the desired microspheres. In view of preventing formation of undesirable aggregate and for improving encapsulation efficiency of the medicament into microspheres, the preferred ratio is in the range of 10:90 to 50:50 by weight, more preferably in the range of 20:80 to 40:60 by weight.

In addition, the homogeneous mixture may contain an emulsion stabilizer. The emulsion stabilizer includes, for example, polyvinyl alcohol, polyvinylpyrrolidone, methylcellulose, hydroxypropylcellulose, gum arabic, chitosan, gelatin, serum albumin, surfactants, etc. Among these, preferable ones are polyvinyl alcohol, polyvinylpyrrolidone, methylcellulose, hydroxypropylcellulose, etc. The emulsion stabilizer may be used in a concentration of 0.001 to 10 % by weight, preferably 0.01 to 2 % by weight.

Solvent A is partially miscible with the homogeneous mixture containing Solvent B and Solvent C. However, owing to the limited dissolution rate thereof, there is formed an emulsion wherein a polymer solution containing a medicament, a biodegradable polymer and Solvent A forms a dispersed phase, and the homogenous mixture forms a continuous phase. Simultaneously or synchronously with the formation of the emulsion, Solvent A is gradually removed from the polymer solution into the homogeneous mixture and thereby the formation of microspheres starts.

The mixing velocity of Solvent A in the polymer solution into the homogeneous mixture can be controlled by changing the ratio by weight of Solvent B and Solvent C in the homogeneous mixture, and the higher the ratio of Solvent C is, the lower the mixing velocity thereof becomes, and the lower the ratio of Solvent C is, the higher the mixing velocity of Solvent A becomes.

Furthermore, by adding previously a small amount of Solvent A into the homogeneous mixture, the mixing velocity of Solvent A in the polymer solution into the homogeneous mixture can possibly be slowed down, and the amount of Solvent A to be added into the homogeneous mixture is not specifically limited as far as Solvent A can be homogeneously miscible in the homogeneous mixture, and it is preferably in the range of not more than 30 % by weight, especially preferably in the range of not more than 20 % by weight.

Preferable ratio of the polymer solution and the homogeneous mixture varies depending on the ratio by weight of Solvent B and Solvent C in the homogeneous mixture and other factors. It may be in the range of 1:1 to 1:1000 by weight, preferably in the range of 1:2 to 1:200 by weight, especially preferably in the range of 1:3 to 1:75 by weight.

From the efficiency aspect of the removal of Solvent A from the dispersed phase after emulsification, the amount of Solvent A in the polymer solution is preferably not more than the maximum amount being miscible with the homogeneous mixture, especially in the range of about 1 to 80 % by weight of the maximum miscible amount.

The emulsification temperature is not specifically limited, but it is preferably carried out at a temperature as low as possible in case that a medicament to be used is unstable to heat. When the emulsification is carried out at a low temperature, especially at a temperature of below 0°C, it is preferable that the ratio by weight of the homogeneous mixture to the polymer solution is lowered within the above-mentioned range, or a small amount of Solvent A is previously added to the homogeneous mixture in order to avoid the precipitation of the biodegradable polymer prior to the emulsion formation. Specifically, it is more preferable to add a small amount of Solvent A into the homogeneous mixture, and the amount of Solvent A to be added is within the above range.

The emulsification of the polymer solution into the homogeneous mixture can easily be carried out by adding the polymer solution into the homogeneous mixture under stirring by using a known emulsification apparatus, for example, a propeller mixer, a turbine impeller mixer, a high-pressure emulsifier, ultrasonic dispersion mixer, a static mixer, etc. The duration necessary for emulsification depends on an emulsification apparatus, a volume of solutions, etc., but it usually takes around 1 to 10 minutes.

The emulsification can also preferably be done by a method such as membrane emulsification, spraying, etc.

For emulsifying by the membrane emulsification method, a porous membrane is set between the polymer solution and the homogeneous mixture, followed by giving a pressure onto the polymer solution so as to extrude the polymer solution into the homogeneous mixture through the pores of porous membrane. If necessary, the homogeneous mixture may be stirred. The porous membrane may have various forms such as plane, tubular, spherical, etc. For example, when a tubular porous membrane is used, it can be done according to either one of the following methods; (i) a method comprising introducing a polymer solution within the inner hollow part of the tubular porous membrane and extruding the polymer solution into the homogeneous mixture existing outside of the tubular porous membrane; and (ii) a method comprising extruding the polymer solution existing outside of the tubular porous membrane into the homogeneous mixture introduced within the inner hollow part of the tubular porous membrane (as reported in e.g., Journal of Microencapsulation, 11(2): 171-178 (1994)).

The porous membranes are preferably porous ceramics, porous glass, etc. The porous ceramics include alumina, zirconia, zeolite, etc., and porous glass includes porous silica glass as disclosed in US Patent No. 2,106,744 and US Patent No. 2,215,039, shirasu (volcanic ash) porous glass as disclosed in US Patent No. 4,657,875, etc. Among these, porous glass is especially preferable.

The porous membrane may be subjected to chemical modification of surface for making its surface hydrophilic or hydrophobic, or for introducing various functional groups to the surface. One of specific examples includes hydrophobic porous glass produced by treating with octadecyltrichlorosilane and trimethylsilane.

The porous membrane may have a pore diameter of in the range of 0.2 to 300 µm, preferably be in the range of 4 to 50 µm.

The extrusion velocity of the polymer solution into the homogeneous mixture depends on the kinds and concentration of the biodegradable polymer in the polymer solution, a composition of the homogeneous mixture, a pore diameter of the porous membrane, etc., but may be limited so that the extrusion amount of the polymer solution is in the range of 5 to 500 ml per hour per 1 m² of the porous membrane.

For emulsification by spraying, the polymer solution is sprayed into the homogeneous mixture by using a known spraying appliance, during which the homogeneous mixture may be stirred, if necessary. The spraying appliance includes, for example, air nozzle, pressure nozzle, ultrasonic nozzle, rotary atomizer, etc.

According to the method of the present invention, since the emulsification is carried out by adding a polymer solution into a homogeneous mixture, as compared with the method wherein a homogeneous mixture is added into a polymer solution to cause phase inversion, not only a high yield of microspheres but also the increased encapsulation efficiency of the medicament into the microspheres and the suppression of the initial burst from the microspheres can be achieved.

After emulsification, the resulting emulsion is fluidized by a suitable method to remove residual Solvent A from the dispersed phase. By this method, Solvent A in the dispersed phase is removed into the continuous phase, and the biodegradable polymer in the dispersed phase completely solidifies to give microspheres.

The method of fluidizing the emulsion can be carried out by circulation or stirring. The circulation may be done by withdrawing a portion of the emulsion from a lower part of emulsion using a pump and returning the portion onto a upper part of the emulsion through a pipe. In addition, the stirring may be done by a conventional stirring method using a stirrer blade, magnetic stirrer, etc.

Besides, after emulsifying, the volume of the continuous phase in the emulsion once formed may be increased, then the removal of Solvent A from the dispersed phase is accelerated. In addition, by increasing of the volume of continuous phase, Solvent A removed into the continuous phase during emulsification may be diluted, and thereby the undesirable adverse effects of Solvent A in the continuous phase on microspheres on the formation stage can be prevented.

The volume of the continuous phase may be increased by mixing the emulsion with a separately prepared solvent for increasing the volume.

The solvent for increasing the volume may be Solvent B or a mixture of Solvent B and Solvent C, and the kinds of Solvent B, Solvent C used as a solvent for increasing the volume may be any one which can be used in the homogeneous mixture, but is not necessarily the same one as used as Solvent B or Solvent C in the homogeneous mixture. Examples of Solvent B and Solvent C used as a solvent for increasing the volume include, but not limited thereto, a monovalent alcohol having 1 to 4 carbon atoms and water as Solvent B, and glycerin as Solvent C, etc. When a medicament used is unstable to heat, Solvent B as a solvent for increasing the volume is a monovalent alcohol such as ethanol, etc. so that it is possible to efficiently remove Solvent A even at a low temperature (e.g., below 0°C).

For increasing the volume, a separately prepared solvent for increasing the volume may be added to the emulsion, or the emulsion is added into a separately solvent for increasing the volume.

The volume may be increased by mixing them at once, or either in several times or continuously.

For improving the efficiency of removal of Solvent A from the dispersed phase by increasing the volume thereof, the increasing of volume is carried out in such a manner that the ratio of Solvent B in the continuous phase of the emulsion after increasing is preferably larger than the ratio of Solvent B in the continuous phase of the emulsion prior to increasing, and more preferably the ratio of Solvent A in the continuous phase after increasing is 0 to 70 % larger than that prior to increasing. Further, the volume of the continuous phase after increasing is preferably 2 to 100 times larger than that prior to increasing.

The time necessary for removal of Solvent A after the emulsification may be within 12 hours, and it is possible to shorten the time by increasing the volume of continuous phase in the emulsion.

In addition, by warming or decompressing the emulsion, the removal of Solvent A can be further accelerated.

The temperature of the emulsion is elevated to 30 to 70°C, and it is not necessary to keep the temperature constant. For example, the temperature can be gradually or stepwise elevated. Further, the pressure may preferably be reduced to 5 to 80 kPa by using a suitable vacuum apparatus.

Further, since Solvent A contained in the dispersed phase of emulsion is removed into the continuous phase, the production of microspheres according to the present invention may be carried out in either (a) an open system in which Solvent A removed from the dispersed phase to the continuous phase can be evaporated out of the apparatus for producing microspheres; or (b) a closed system in which Solvent A can not be evaporated out of the apparatus. However, it is more preferable to do in a closed system in order to prevent the bad influence of Solvent A onto environment and to recover and reuse Solvent A. When producing microspheres in a closed system, it is preferable to trap and recover Solvent A being evaporated from the continuous phase.

The recovery of Solvent A can be easily performed by cooling vapor containing Solvent A for condensation or by introducing the vapor to porous particles to adsorb Solvent A.

The microspheres thus produced can be recovered by collecting them by centrifugation, filtration with a filter, etc., followed by washing with water, etc., if necessary, and by complete removal of moisture by air drying, vacuum drying, or lyophilization.

When it is necessary to reduce the amount of Solvent A remained in the microspheres as low as possible, for example, to 5000 ppm or below on the basis of the weight of the microspheres, the microspheres thus produced are dispersed again in water and stirred for a period of 3 minutes to 12 hours, preferably for a period of 5 minutes to 5 hours, and then followed by collecting again said microspheres, which are further washed and dried if necessary.

Depending on formulation to be selected, the microspheres after washing are suspended in a suitable solution, then lyophilized to give a final form of objective formulation.

The microspheres prepared by the above methods may have a particle size of 1-1000 µm in average. Microspheres wherein more than 70 % of particles have the size of 20-150 µm may easily be prepared.

The microspheres thus prepared has a high encapsulation efficiency of a medicament regardless of the kind of the medicament. The dissolution pattern may be a type of release in zero-order as seen in the following Examples.

The microspheres prepared by the method of this invention may easily be administered by injection or as an implant, intramuscularly, subcutaneously, intravenously, intra-organ or intra-articularly, intraperitoneally, intrafocally such as in tumor organ, etc. They may also be used as raw material for preparing various formulations. Such formulations include, for example, injection, oral, transdermal, intrarectal, transnasal, transpulmonary, intrastomatal, or intraophthal formulations.

### EXAMPLES

The present invention is illustrated in more detail by the following Examples and Comparative Experiments.

### Example 1

Acetone (800 mg) was added to poly(lactic-co-glycolic acid) (487.5 mg, molar ratio of lactic acid/glycolic acid: 50:50; molecular weight: 20,000; manufactured by Wako Pure Chemical Industries, Ltd.) (hereinafter, abbreviated to PLGA 5020) and vitamin B₁₂ (12.5 mg, manufactured by Rhone-Poulenc) which was previously pulverized by a jet mill (manufactured by Seishin Enterprise Co. Ltd.) to give a polymer solution, wherein vitamin B₁₂ was in dispersed state. To a glycerin/water mixture (4 g, ratio of glycerin/water: 70:30 by weight) containing polyvinyl alcohol (1.0 % by weight) was added the polymer solution by using a Pasteur pipette under stirring at 1500 rpm by a propeller mixer (Three One Motor BL 3000, manufactured by Heidon) at room temperature for emulsification for 3 minutes to give an emulsion comprising the polymer solution as a dispersed phase and the glycerin/water mixture as a continuous phase. This emulsion was added to a glycerin/water mixture (14 g, ratio of glycerin/water: 70:30 by weight). After sealing the vessel, the mixture was stirred for 3 hour with a magnetic stirrer to remove acetone from the dispersed phase to give a dispersion of microspheres. This dispersion was passed through a filter of 150 µm, and the microspheres were collected by filtration using a filter of 20 µm, and lyophilized to recover the microspheres. The yield of the microspheres (ratio of the amount of the recovered microspheres to the amount of the starting polymer and the medicament used) was 79.5 %.

The mean particle size of the recovered microspheres was 47.0 µm, and the encapsulation efficiency of the medicament was 81.0 % (the amount of vitamin B₁₂ was measured by a spectrophotometer, Shimadzu UV-2500PC).

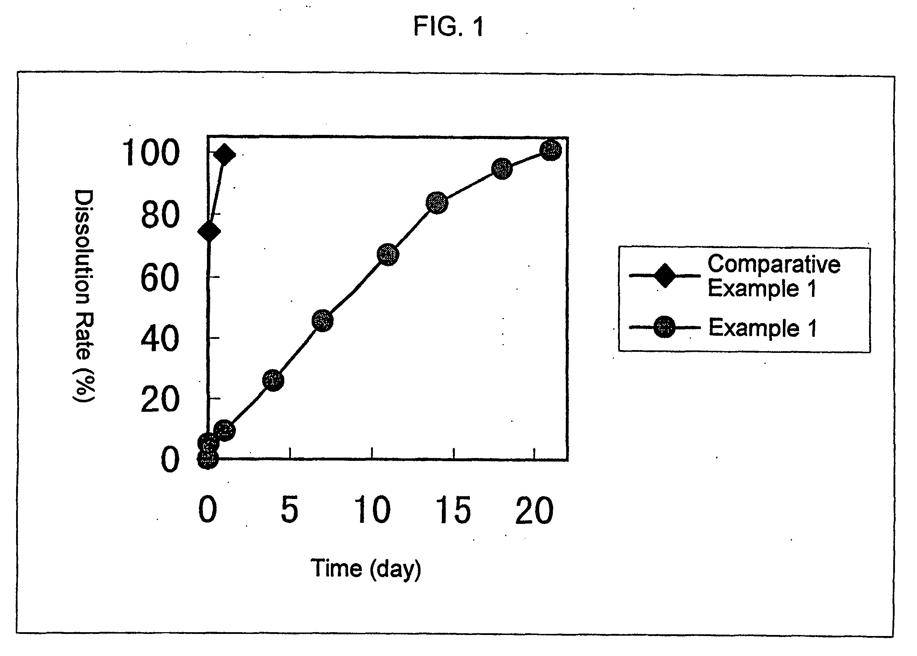

The dissolution test *in vitro* (solvent for dissolution: a 9.6 mM phosphate buffered physiological saline (pH 7.4); dissolution test machine: Taitec rotary fermenter RT50 (stirring intensity: 25 rpm); 37°C) of the microspheres thus obtained showed that vitamin B₁₂ was released from the microspheres at a constant rate over 21 days. The initial burst rate (i.e., the dissolution rate at one hour after the start of experiment) was merely 5.2 % (Fig. 1).

### Comparative Example 1

A polymer solution was prepared in a similar manner as in Example 1. To the polymer solution, a glycerin/water mixture (4 g, ratio of glycerin/water: 70:30 by weight) containing polyvinyl alcohol (1.0 % by weight) was added with a Pasteur pipette under stirring at 1500 rpm by a propeller mixer (Three One Motor BL 3000, manufactured by Heidon) at room temperature for emulsification for 3 minutes. The phase inversion of the emulsion was observed to give an emulsion comprising the polymer solution as a dispersed phase and the glycerin/water mixture as a continuous phase. This emulsion was added to a glycerin/water mixture (14 g, ratio of glycerin/water: 70:30 by weight). After sealing the vessel, the mixture was stirred for 3 hours with a magnetic stirrer to remove acetone from the dispersed phase of emulsion to give a dispersion of microspheres. This dispersion was passed through a filter of 150 µm, and the microspheres were collected by filtration using a filter of 20 µm, and lyophilized to recover the microspheres.

The mean particle size of the recovered microspheres was 37.7 µm, and the encapsulation efficiency of the medicament was merely 27.7 %.

The dissolution test *in vitro* of the microspheres thus obtained showed that most of the medicament was released within one hour and the initial burst rate was as high as 74.1 % (Fig. 1).

From the above results, it was shown that by addition of the glycerin/water mixture into the polymer solution, the encapsulation efficiency of the medicament was lowered, and the initial burst rate was increased.

### Comparative Example 2

A polymer solution was prepared in a similar manner as in Example 1. The polymer solution was added to a saturated aqueous sucrose solution (sucrose concentration: about 65 % by weight) containing polyvinyl alcohol (1.0 % by weight) by using a Pasteur pipette under stirring at 1500 rpm by a propeller mixer (Three One Motor BL 3000, manufactured by Heidon) at room temperature for emulsification for 3 minutes to give an emulsion comprising the polymer solution as a dispersed phase and the saturated sucrose solution as a continuous phase. This emulsion was added to a glycerin/water mixture (14 g, ratio of glycerin/water: 50:50 by weight). After sealing the vessel, the mixture was stirred for 3 hours by a magnetic stirrer to remove acetone from the dispersed phase of the emulsion to give a dispersion of fine particles. This dispersion was passed through a filter of 150 µm, and the fine particles were collected by filtration using a filter of 20 µm, and lyophilized to recover fine particles.

The fine particles however were fibrous but not spherical microspheres, and the recovery rate was merely 3.5 %.

Therefore, it was shown that microspheres cannot be obtained by emulsifying a polymer solution in a saturated aqueous sugar (sucrose) solution.

### Comparative Example 3

The same procedures of Comparative Example 2 were repeated except that a saturated aqueous glucose solution (glucose concentration: about 50 % by weight) containing polyvinyl alcohol (1.0 % by weight) was used instead of a saturated aqueous sucrose solution (sucrose concentration: about 65 % by weight) containing polyvinyl alcohol (1.0 % by weight) to give fine particles.

However, the fine particles were fibrous but not spherical microspheres, and the recovery rate was merely 5.1 %.

Therefore, it was shown that microspheres cannot be obtained by emulsification of the polymer solution in a saturated aqueous sugar (glucose) solution.

### Comparative Example 4

The same procedures of Comparative Example 2 were repeated except that a saturated aqueous mannitol solution (mannitol concentration: about 15 % by weight) containing polyvinyl alcohol (1.0 % by weight) was used instead of a saturated aqueous sucrose solution (sucrose concentration: about 65 % by weight) containing polyvinyl alcohol (1.0 % by weight) to give fine particles.

However, the particles were fibrous but not spherical microspheres, and the recovery rate was merely 0.7 %.

Therefore, it was shown that microspheres cannot be obtained by emulsification of the polymer solution in a saturated aqueous sugar (mannitol) solution.

### Example 2

Acetone (800 mg) was added to PLGA 5020 (487.5 mg) and vitamin B₁₂ (12.5 mg) which was previously pulverized by a jet mill (manufactured by Seishin Enterprise Co. Ltd.) to prepare a polymer solution, wherein vitamin B₁₂ was in dispersed state. The polymer solution was added to a glycerine/water mixture (6 g, ratio of glycerin/water: 70:30 by weight) containing polyvinyl alcohol (0.3 % by weight) with a Pasteur pipette under stirring at 2500 rpm by an emulsifier (POLYTRON®, manufactured by Kinematica AG Littau) at 15°C for emulsification for 3 minutes to give an emulsion comprising the polymer solution as a dispersed phase and the glycerin/water mixture as a continuous phase. This emulsion was added to a glycerin/water mixture (14 g, ratio of glycerin/water: 50:50 by weight) and stirred for 2.5 hours by a magnetic stirrer. To the emulsion was added water (10 ml), and the emulsion was stirred for 0.5 hour to remove acetone from the dispersed phase of emulsion to give a dispersion of microspheres. This dispersion was passed through a filter of 150 µm, and the microspheres were collected by filtration using a filter of 20 µm, and lyophilized to recover the microspheres.

The mean particle size of the recovered microspheres was 62.7 µm, and the encapsulation efficiency of the medicament was 61.3 %.

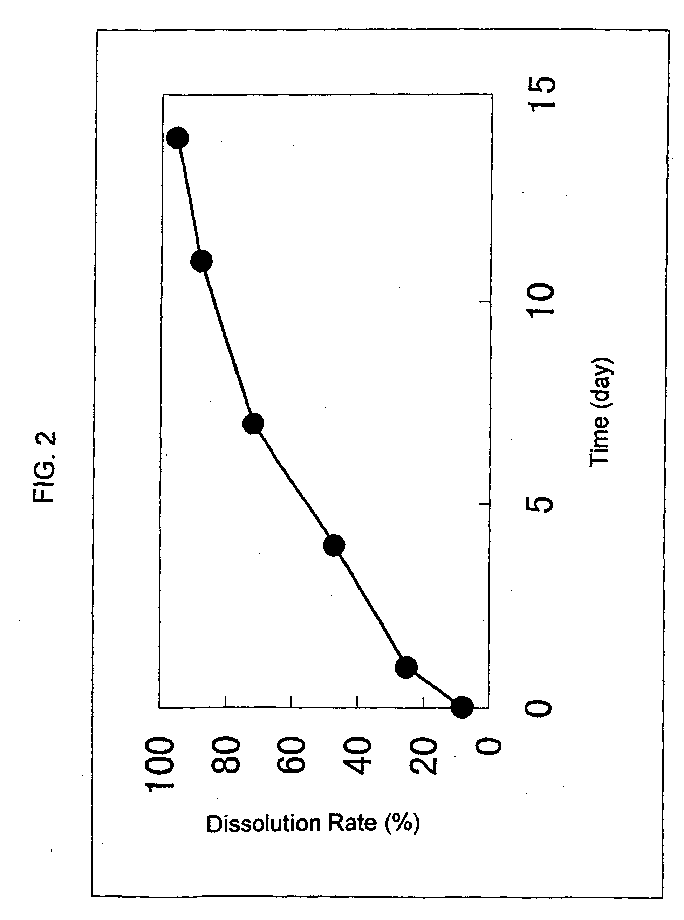

The dissolution test *in vitro* (solvent for dissolution: a 9.6 mM phosphate buffered physiological saline (pH 7.4; 37°C) of the microspheres thus obtained showed that vitamin B₁₂ was released at a constant rate over 14 days (Fig. 2).

### Example 3

In a manner as disclosed in JP-A-11-302156, bovine serum albumin (1 g, manufactured by Sigma, hereinafter abbreviated to BSA), and polyethylene glycol 6000 (2 g, manufactured by Wako Pure Chemical Industries, Ltd.) were dissolved in water (100 mL), and the resulting solution was lyophilized. The resulting solid was washed with a mixture of acetone and methylene chloride (volume ratio of acetone and methylene chloride: 3:1) to remove polyethylene glycol, and dried under reduced pressure for one hour to give BSA fine particles having a mean particle size of 1 µm.

The same procedures of Example 2 were repeated except that a polymer solution (wherein BSA was in dispersed state) was prepared by using fine particles of BSA as described above instead of previously pulverized vitamin B₁₂ and microspheres were collected by centrifugation (2000 rpm, 5 minutes) twice instead of filtration using a filter of 20 µm.

The mean particle size of the recovered microspheres was 14.3 µm and the encapsulation efficiency of the medicament was 74.8 % (the amount of BSA was measured by Micro BCA protein assay kit, PIERCE).

### Example 4

The same procedures of Example 2 were repeated except that a polymer solution (wherein estrone was in solution state) was prepared by using estrone instead of previously pulverized vitamin B₁₂ and microspheres were collected by centrifugation (2000 rpm, 5 minutes) twice instead of by filtration using a filter of 20 µm.

The mean particle size of the recovered microspheres was 22.4 µm and the encapsulation efficiency of the medicament was nearly 100 % (the amount of estrone was measured by HPLC method).

### Example 5

The same procedures of Example 2 were repeated except that a glycerin/ethanol mixture (ratio of glycerin/ethanol: 80:20 by weight) containing hydroxypropyl cellulose (1 % by weight; HPC-L, manufactured by Nippon Soda) was used instead of a glycerin/water mixture (ratio of glycerin/water: 70:30 by weight) containing polyvinyl alcohol (0.3 % by weight), and the setting rotation of POLYTRON® was 4000 rpm to give microspheres having a mean particle size of 50.8 µm.

### Example 6

The same procedures of Example 2 were repeated except that a polymer solution (wherein vitamin B₁₂ was in dispersed state) was prepared by using tetrahydrofuran instead of acetone, and microspheres were collected by centrifugation (2000 rpm, 5 minutes) twice instead of by filtration using a filter of 20 µm to give microspheres having a mean particle size of 13.8 µm.

### Example 7

The same procedures of Example 2 were repeated except that a polymer solution was prepared by employing polylactic acid (molecular weight: 20000, manufactured by Wako Pure Chemical Industries, Ltd.) and BSA being micronized according to a method as disclosed in JP-A-11-302156 (wherein BSA was in a dispersed state) instead of PLGA 5020 and vitamin B₁₂ respectively, and a propeller mixer (1500 rpm; Three One Motor BL 3000, manufactured by Heidon) was used instead of POLYTRON® to give microspheres.

The mean particle size of the recovered microspheres was 76.1 µm and the encapsulation efficiency of the medicament was 78.9 %.

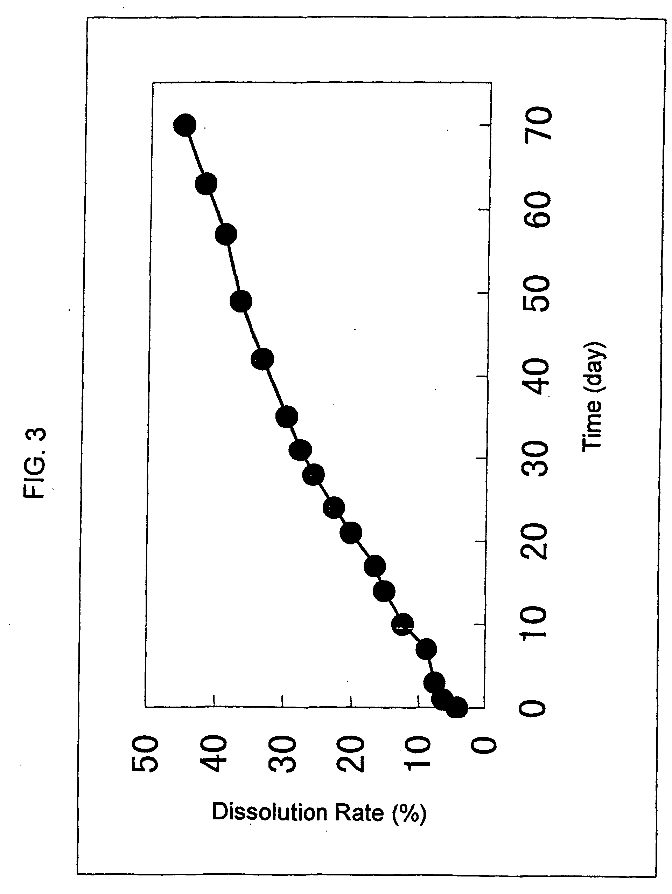

The results of the dissolution test *in vitro* of the microspheres are shown in Fig. 3.

### Example 8

Acetone (700 mg) and water (100 mg) were added to PLGA 5020 (487.5 mg) and taltirelin hydrate (12.5 mg) to give a polymer solution (wherein taltirelin hydrate was in a dissolved state). The polymer solution was added to a glycerin/water mixture (4 g, ratio of glycerin/water: 70:30 by weight) containing polyvinyl alcohol (1.0 % by weight) with a Pasteur pipette under stirring at 1500 rpm by a propeller mixer (Three One Motor BL 3000, manufactured by Heidon) at room temperature for emulsification for 3 minutes to give an emulsion comprising the polymer solution as a dispersed phase and the glycerin-water mixture as a continuous phase. This emulsion was added into a glycerin/water mixture (14 g, ratio of glycerin/water: 70:30 by weight) and the resulting mixture was stirred for 3 hours with a magnetic stirrer to remove acetone from the dispersed phase of emulsion to give a dispersion of microspheres. This dispersion was passed through a filter of 150 µm, and microspheres were collected by filtration using a filter of 20 µm, and lyophilized to recover microspheres.

The mean particle size of the recovered microspheres was 76.0 µm and the encapsulation efficiency of the medicament was 46.1 % (the amount of taltirelin was measured by HPLC method).

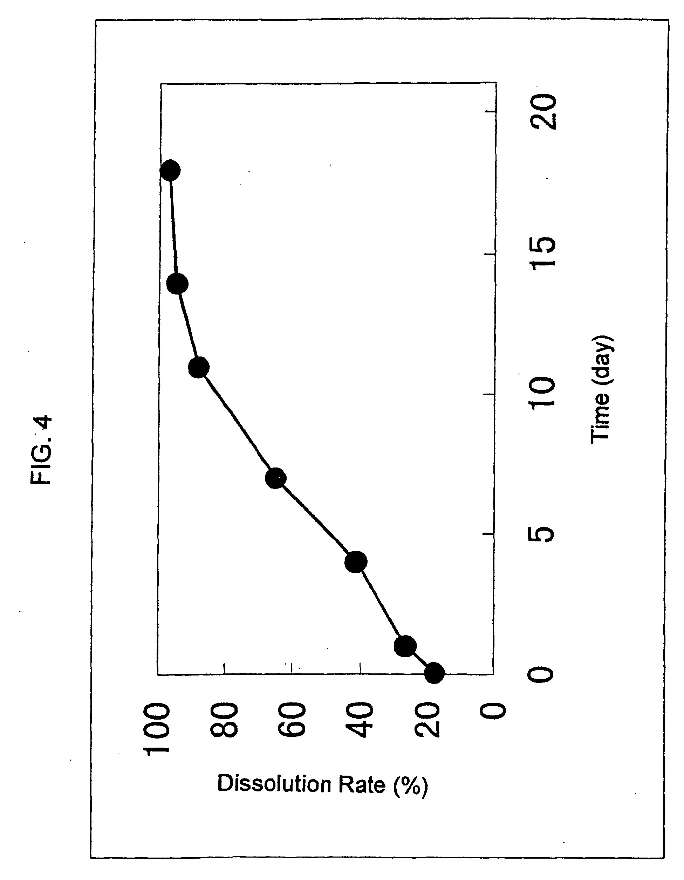

The results of the dissolution test *in vitro* of microspheres are shown in Fig. 4.

### Example 9

Acetone (800 mg) was added to PLGA 5020 (487.5 mg) and vitamin B₁₂ (12.5 mg) which was previously pulverized with a jet mill (manufactured by Seishin Enterprise Co. Ltd.) to give a polymer solution (wherein vitamin B₁₂ was in a dispersed state). The polymer solution was added to a glycerin/water mixture (4 g, ratio of glycein/water: 70:30 by weight) containing polyvinyl alcohol (0.5 % by weight) with a Pasteur pipette under stirring at 500 rpm by a Ramond stirrer (type ST02, manufactured by EST Kankyo Kagaku Kogyo) at room temperature for emulsification for 3 minutes to give an emulsion comprising the polymer solution as a dispersed phase and the glycerin/water mixture as a continuous phase. This emulsion was added into a glycerin/water mixture (14 g, ratio of glycerin/water: 50:50 by weight) and the resulting mixture was stirred for 0.5 hours and stirred at 50°C for 2.5 hours with a magnetic stirrer to remove acetone from the dispersed phase of emulsion to give a dispersion of microspheres. This dispersion was passed through a filter of 150 µm, and microspheres were collected by filtration using a filter of 20 µm, and lyophilized to recover microspheres.

The mean particle size of the recovered microspheres was 55.1 µm and the encapsulation efficiency of the medicament was 77.4 %.

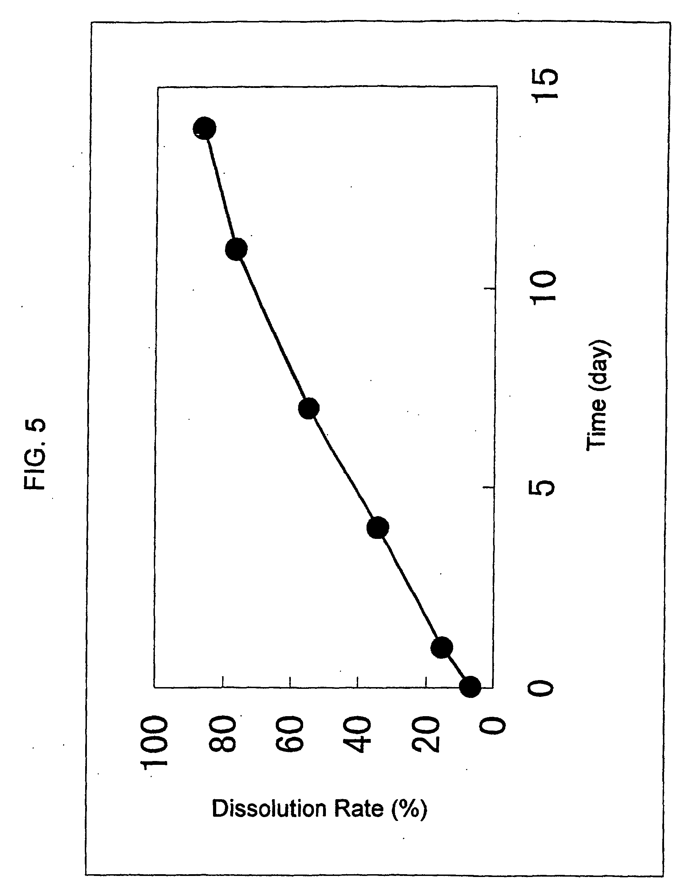

The results of the dissolution test *in vitro* of the microspheres are shown in Fig. 5.

### Example 10

BSA (1 g) and PEG 6000 (3 g, polyethylene glycol 6000, manufactured by Katayama Chemical Inc.) were dissolved in water (200 ml), and the solution was frozen at -20°C. Acetone (500 ml) was added to the resulting frozen product, and the mixture was stirred at 500 rpm by a propeller mixer (Three One Motor BL 3000, manufactured by Heidon) to dissolve PEG 6000 and ice in acetone to give a dispersion of BSA fine particles. This dispersion was centrifuged at 2000 rpm for 5 minutes to remove the supernatant, and the BSA fine particles were washed twice with acetone (50 ml), dried under reduced pressure overnight to give BSA fine particles having a mean particle size of 3.72 µm.

Acetone (1500 mg) was added to the resulting BSA fine particles (25 mg) and Resomer RG503H (475 mg, poly(lactic-co-glycolic acid), molar ratio of lactic acid/glycolic acid: 50:50, molecular weight: 33000, manufactured by Boehringer) to give a polymer solution (wherein BSA was in dispersed state). The polymer solution was added to a mixture of glycerin/water mixture (8 g, ratio of glycerin/water: 70:30 by weight) containing polyvinyl alcohol (0.5 % by weight) and acetone (1 g) with a Pasteur pipette under stirring at 500 rpm by a propeller mixer (Three One Motor BL 3000, manufactured by Heidon) at -20°C for emulsification for 3 minutes to give an emulsion comprising the polymer solution as a dispersed phase and the glycerin/water mixture as a continuous phase. This emulsion was added to ethanol (30 ml) a - 20°C, and after sealing the vessel, the mixture was stirred for 3 hour with a magnetic stirrer to remove acetone from the dispersed phase of emulsion to give a dispersion of microspheres. This dispersion was passed through a filter of 150 µm, and microspheres were collected by filtration using a filter of 20 µm, washed with water, and lyophilized to recover microspheres. The yield of the microspheres was 74.5 %.

The encapsulation efficiency of the medicament in the resulting microspheres was 68.6 %.

### Example 11

The microsphere dispersion obtained in a similar manner as in Example 10 was passed through a filter of 150 µm, and microspheres were collected by filtration using a filter of 20 µm, dispersed again in water (10 ml), and the resulting dispersion was stirred at room temperature for 2.5 hours in a closed system. After the stirring is completed, microspheres were collected again by filtration using a filter of 20 µm, and lyophilized to recover microspheres.

The resulting microspheres were dissolved in dioxane, and the content of acetone remained in the microspheres was measured by gas chromatography. As a result, it was not more than 500 ppm.

### Example 12

BSA (2 g) and PEG 20000 (6 g, polyethylene glycol 20000, manufactured by Katayama Chemical Inc.) were dissolved in water (200 ml), and the solution was frozen at -80°C. Acetone (500 ml) was added to the resulting frozen product, and the mixture was stirred at 500 rpm by a propeller mixer (Three One Motor BL 3000, manufactured by Heidon) to dissolve PEG 20000 and ice in acetone to give a dispersion of BSA fine particles. This dispersion was centrifuged at 2000 rpm for 5 minutes, and the supernatant was removed. The BSA fine particles were washed twice with acetone (50 ml), and dried under reduced pressure overnight to give BSA fine particles having a mean particle size of 2.04 µm.

Acetone (800 mg) was added to the resulting BSA fine particles (12.5 mg) and PLGA 5020 (487.5 mg) to give a polymer solution wherein BSA was in dispersed state. The polymer solution was added to a mixture of a glycerine/water mixture (4 g, ratio of glycerine/water: 70:30 by weight) containing polyvinyl alcohol (0.5 % by weight) and acetone (0.5 g) with a Pasteur pipette at -20°C under stirring at 500 rpm by a propeller mixer (Three One Motor BL 3000, manufactured by Heidon) for emulsification for 3 minutes to give an emulsion comprising the polymer solution as a dispersed phase and the glycerin/water mixture as a continuous phase. This emulsion was added to ethanol (7.5 ml) at -20°C, and after sealing the vessel, the mixture was stirred with a magnetic stirrer for 15 minutes to remove the acetone from the dispersed phase of emulsion to give a dispersion of microspheres. This dispersion was passed through a filter of 150 µm, and the microspheres were collected by filtration using a filter of 20 µm. The microspheres thus collected were dispersed again in water (10 ml), and stirred at room temperature for one hour in a closed system. After the stirring was completed, microspheres were collected again by filtration using a filter of 20 µm, and lyophilized to recover microspheres.

### Example 13

The same procedures of Example 12 were repeated except that a mixture of a glycerin/water mixture (4 g, ratio of glycerin/water: 70:30 by weight) containing polyvinyl alcohol (0.5 % by weight) and tetrahydrofuran (0.5 g) was used instead of a mixture of a glycerin/water mixture (4 g, ratio of glycerin/water: 70:30 by weight) containing polyvinyl alcohol (0.5 % by weight) and acetone (0.5 g) to give microspheres.

### Example 14

The same procedures of Example 12 were repeated except that a mixture of glycerin/water mixture (4 g, ratio of glycerin/water: 70:30 by weight) containing polyvinyl alcohol (0.5 % by weight) and acetonitrile (0.5 g) was used instead of a mixture of a glycerin/water mixture (4 g, ratio of glycerin/water: 70:30 by weight) containing polyvinyl alcohol (0.5 % by weight) and acetone (0.5 g) to give microspheres.

### Example 15

The dispersion of microspheres obtained in a similar manner as in Example 12 was passed through a filter of 150 µm, and microsphers were collected by filtration using a filter of 20 µm, dispersed again in water (1 ml), and stirred at 4°C for one hour in a closed system. After the stirring was completed, the mixture was lyophilized to recover the microspheres.

### INDUSTRIAL APPLICABILITY

According to the method of the present invention, microspheres can be prepared by using a water-miscible organic solvent, especially by using acetone, tetrahydrofuran, etc. being less harmful to human body or environment.

Furthermore, according to the method of the present invention, either water soluble medicaments or fat soluble medicaments can be incorporated in microspheres at a high encapsulation efficiency.

## Claims

1. A process for producing microspheres, which comprises adding a polymer solution containing a medicament, a biodegradable polymer and a good solvent for said polymer which solvent is miscible with water (Solvent A) to a homogeneous mixture of a poor solvent for said polymer which solvent is miscible with said Solvent A (Solvent B) and a poor solvent for said polymer which solvent is immiscible with said Solvent A (Solvent C), emulsifying the mixture to prepare an emulsion wherein the polymer solution forms a dispersed phase and the homogeneous mixture forms a continuous phase, and then removing Solvent A from the dispersed phase.

2. The process according to claim 1, wherein the biodegradable polymer is a polyester of a hydroxyfatty acid.

3. The process according to claim 1, wherein the biodegradable polymer is one or more members selected from polylactic acid, a copolymer of lactic acid - glycolic acid, and a copolymer of 2-hydroxybutyric acid - glycolic acid.

4. The process according to any one of claims 1 to 3, wherein Solvent A is one or more members of the solvents selected from a group consisting of acetone, tetrahydrofuran, acetonitrile, dimethylformamide, dimethylsulfoxide, dioxane, diglyme and ethyleneglycol dimethyl ether, Solvent B is one or more members of the solvents selected from the group consisting of water and a monovalent alcohol having 1 to 4 carbon atoms, and Solvent C is glycerine.

5. The process according to claim 4, wherein the monovalent alcohol having 1 to 4 carbon atoms is methanol, ethanol, n-propanol, isopropanol, n-butanol, isobutanol, sec-butanol, or tert-butanol.

6. The process according to claim 4, wherein Solvent A is acetone, Solvent B is water, and Solvent C is glycerine.

7. The process according to any one of claims 1 to 6, wherein the ratio of Solvent B and Solvent C in the homogeneous mixture is in the range of 5:95 to 75:25 by weight.

8. The process according to any one of claims 1 to 7, wherein the amount of Solvent A is not more than the maximum amount of Solvent A being miscible with the homogenous mixture of Solvent B and Solvent C.

9. The process according to any one of claims 1 to 8, wherein the polymer solution contains Solvent B.

10. The process according to claim 9, wherein the polymer solution has a content of Solvent B in the range of 0.001 to 50 % by weight.

11. The process according to any one of claims 1 to 10, wherein the homogeneous mixture contains an emulsion stabilizer.

12. The process according to any one of claims 1 to 11, wherein the homogeneous mixture contains Solvent A.

13. The process according to claim 12, where the emulsification procedure is carried out below 0°C.

14. The process according to any one of claims 1 to 13, wherein the ratio of the polymer solution and the homogeneous mixture is in the range of 1:1 to 1:1000 by weight.

15. The process according to any one of claims 1 to 14, wherein after emulsifying the amount of the continuous phase is increased by mixing the resulting emulsion and a separately prepared increasing solvent, and Solvent A is removed.

16. The process according to claim 15, wherein the increasing solvent is Solvent B or a mixture of Solvent B and Solvent C.

17. The process according to claim 16, wherein Solvent B used as the increasing solvent is one or more solvents selected from water and a monovalent alcohol having 1 to 4 carbon atoms, and Solvent C used as the increasing solvent is glycerine.

18. The process according to claim 16, wherein Solvent B used as the increasing solvent is a monovalent alcohol having 1 to 4 carbon atoms, and the removal of Solvent A is carried out below 0°C.

19. The process according to any one of claims 15 to 18, wherein the ratio of Solvent B in the continuous phase of the emulsion after increasing thereof is the same as or larger than the ratio of Solvent B before increasing.

20. The process according to any one of claims 15 to 19, wherein the continuous phase after increasing thereof has a volume of 2 to 100 times larger than that before increasing.

21. The process according to any one of claims 1 to 12, 14 to 17, 19 and 20, wherein the removal of Solvent A is carried out by warming the emulsion.

22. The process according to claim 21, wherein the warming is carried out at a temperature of 30 to 70°C.

23. The process according to any one of claims 1 to 22, wherein the removal of Solvent A is carried out by placing the emulsion under reduced pressure after emulsifying step.

24. The process according to claim 23, wherein the pressure after reduced pressure is in the range of 5 to 80 kPa.

25. The process according to any one of claims 1 to 24, wherein the removal of Solvent A is carried out in a closed system.

26. A process for removing Solvent A remained in the microspheres prepared by the process as set forth in one of claims 1 to 25, which comprising re-dispersing said microspheres in water, and stirring the resulting mixture.
